(19) Europäisches Patentamt
European Patent Office
Office européen des brevets

(11) Veröffentlichungsnummer: **0 353 670 B1**

# (12) EUROPÄISCHE PATENTSCHRIFT

(45) Veröffentlichungstag der Patentschrift: **29.09.93**

(51) Int. Cl.5: **C07D 319/12**, C07D 319/04, C07D 321/08, C07D 321/12, C07D 495/10, C07C 31/20, C07C 43/13

(21) Anmeldenummer: **89114033.7**

(22) Anmeldetag: **29.07.89**

## (54) Verfahren zur Herstellung cyclischer 6- bis 8-gliedriger Vinylen-1,2-dioxyverbindungen.

(30) Priorität: **03.08.88 DE 3826303**

(43) Veröffentlichungstag der Anmeldung:
**07.02.90 Patentblatt 90/06**

(45) Bekanntmachung des Hinweises auf die Patenterteilung:
**29.09.93 Patentblatt 93/39**

(84) Benannte Vertragsstaaten:
**BE CH DE FR GB IT LI NL**

(56) Entgegenhaltungen:
**EP-A- 0 044 444**
**CH-A- 643 550**

**CHEMICAL ABSTRACTS, Band 103, Nr. 13, 30. September 1985, Columbus, Ohio, USA KARAEV, S.F.; MAMEDOV, E.A.; GARAEVA, SH. V.: "Intra- molekular cyclization of monopyropargyl ethers of 1,2-propane- and 1,3-butane- diols" Seite 620, Spalte 1, Zusammenfassung-Nr. 104 935v**

(73) Patentinhaber: **BASF Aktiengesellschaft**
**Carl-Bosch-Strasse 38**
**D-67063 Ludwigshafen(DE)**

(72) Erfinder: **Merger, Franz, Dr.**
**Max-Slevogt-Strasse 25**
**D-6710 Frankenthal(DE)**
Erfinder: **Frank, Juergen, Dr.**
**Hirschbrunnenweg 82**
**D-6830 Schwetzingen(DE)**
Erfinder: **Hoelderich, Wolfgang, Dr.**
**Mannheimer Strasse 18 c**
**D-6710 Frankenthal(DE)**
Erfinder: **Dockner, Toni, Dr.**
**Grossgasse 6**
**D-6701 Meckenheim(DE)**
Erfinder: **Sauerwald, Manfred, Dr.**
**Im Langen Satz 2**
**D-6701 Meckenheim(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die vorliegende Erfindung betrifft ein neues und verbessertes Verfahren zur Herstellung von cyclischen 6- bis 8-gliedrigen Vinylen-1,2-dioxyverbindungen durch thermische, säurekatalysierte Ringerweiterung von cyclischen 2-Oxymethyl-1,3-dioxaverbindungen oder deren Derivaten.

Aus J. Het. Chem. 17, 831-832 (1980) ist bekannt, daß man 1,4-Dioxene durch säurekatalysierte Alkoholabspaltung aus 2-Alkoxy-1,4-dioxanen herstellen kann.

Aus Chem. Abstr. 103; 104, 935r, J. Chem. Res. 273 (1977) und J. Org. Chem. 31 (2), 389-91 (1966) sind einige 1,4-Dioxep-2-enverbindungen bekannt, die an der Doppelbindung einen Substituenten tragen.

Der Erfindung lag die Aufgabe zugrunde, einen besseren Zugang zu cyclischen 6- bis 8-gliedrigen Vinylen-1,2-dioxyverbindungen zu finden.

Demgemäß wurde ein neues und verbessertes Verfahren zur Herstellung von cyclischen 6- bis 8-gliedrigen Vinylen-1,2-dioxyverbindungen gefunden, welches dadurch gekennzeichnet ist, daß man cyclische 2-Oxymethyl-1,3-dioxaverbindungen oder deren Derivate in Gegenwart von sauren Katalysatoren bei Temperaturen von 150 bis 450 °C und Drücken von 0,001 bis 50 bar umsetzt.

Die cyclischen 6- bis 8-gliedrigen Vinylen-1,2-dioxyverbindungen I sind nach folgender Methode erhältlich:

Die Umsetzung erfolgt durch Kontakt einer cyclischen 2-Oxymethyl-1,3-dioxaverbindung oder eines Derivates II mit einem sauren Katalysator nach folgender Reaktionsgleichung:

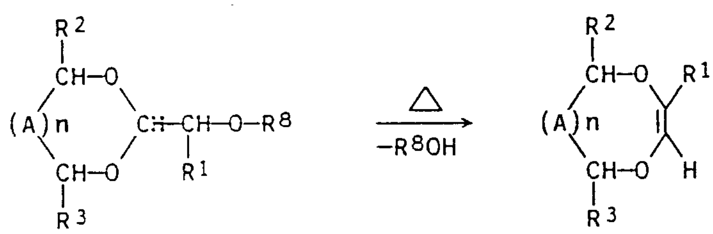


(II) (I)

Der bifunktionelle Rest A bedeute

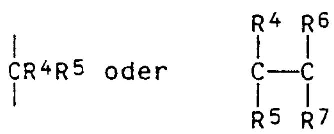

und

der Index n bedeutet 0 oder 1.

Die Reaktion kann sowohl in der Gasphase als auch in der bevorzugten Flüssigphase diskontinuierlich, vorzugsweise semikontinuierlich oder besonders bevorzugt kontinuierlich bei Temperaturen von 150 bis 450 °C und Drücken von 0,001 bis 50 bar durchgeführt werden.

Die Gasphasenreaktion kann beispielsweise bei Temperaturen von 200 bis 450 °C und Drücken von 0,001 bis 50 bar, vorzugsweise bei 250 bis 350 °C und 0,02 bis 25 bar, besonders bevorzugt bei 270 bis 350 °C und 0,05 bis 10 bar durchgeführt werden.

Bei der Umsetzung in der Gasphase hält man eine Katalysatorbelastung von 0,01 bis 5, vorteilhaft von 0,05 bis 2 g, insbesondere 0,1 bis 2 g Ausgangsstoff der Formel II je g Katalysator und Stunde ein (WHSV = g Einsatzgemisch/g Katalysator und Stunde). Die Gasphasenreaktion kann in einem Festbett oder in einem Wirbelbett ausgeführt werden.

Als Katalysatoren für das erfindungsgemäße Verfahren werden saure feste Heterogenkatalysatoren, eingesetzt.

Als Katalysatoren für das erfindungsgemäße Verfahren werden insbesondere acide zeolithische Katalysatoren eingesetzt. Eine Beschreibung der hierfür in Frage kommenden Zeolithe findet sich in DE-A-35 13 725, DE-A-35 46 372, DE-A-36 30 684 und DE-A-36 38 010.

Für das erfindungsgemäße Verfahren in Betracht kommende Katalysatoren sind Zeolithe aus der Mordenit-Gruppe oder engporige Zeolithe vom Erionit- bzw. Chabasit-Typ oder Zeolithe vom Faujasit-Typ, z.B. Y-, X- oder L-Zeolithe. In diese Gruppe von Zeolithen gehören auch die sogenannten "ultrastabile"

Zeolithe des Faujasittyps, d.h. dealuminierte Zeolithe.

Besonders vorteilhaft sind Zeolithe vom Pentasiltyp. Diese Zeolithe können unterschiedliche chemische Zusammensetzung aufweisen. Es handelt sich hierbei um Alumino-, Boro-, Eisen-, Beryllium-, Gallium-, Chrom-, Arsen-, Antimon- und Wismutsilikatzeolithode oder deren Gemische sowie Alumino-, Boro-, Gallium- und Eisengermanatzeolithe oder deren Gemische. Insbesondere eignen sich die Alumino-, Boro- und Eisensilikatzeolithe des Pentasiltyps für das erfindungsgemäße Verfahren.

Zu den verwendbaren siliciumreichen Zeolithen ($SiO_2/Al_2O_3 \geqq 10$) gehören auch die sog. ZSM-Typen, Ferrierit, NU-1 und Silicalit® (ein Molekularsieb, ein sog. Silica Polymorph).

Die so hergestellten Alumino-, Boro- und Eisensilikatzeolithe können nach ihrer Isolierung, mit einem Bindemittel im Verhältnis 90:10 bis 40:60 Gew.% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25:75 bis 90:5, bevorzugt 75:25, Siliciumdioxid, bevorzugt hochdisperses $SiO_2$, Gemische aus hochdispersem $SiO_2$ und hochdispersem $Al_2O_3$, $TiO_2$, $ZrO_2$ sowie Ton.

Man erhält auch vorteilhafte Katalysatoren, wenn der isolierte Alumino-bzw. Borosilikatzeolith direkt nach der Trocknung verformt wird und erst nach der Verformung einer Calcinierung unterworfen wird. Die hergestellten Alumino- und Borosilikatzeolithe können in reiner Form, ohne Binder, als Stränge oder Tabletten eingesetzt werden, wobei als Verstrangungs- oder Peptisierungshilfsmittel z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden.

Liegt der Zeolith aufgrund der Art seiner Herstellung nicht in der katalytisch aktiven, aciden H-Form vor, sondern z.B. in der Na-Form, dann kann diese durch Ionenaustausch z.B. mit Ammoniumionen und anschließende Calcinierung oder durch Behandlung mit Säuren vollkommen oder partiell in die gewünschte H-Form überführt werden.

Wenn bei der erfindungsgemäßen Verwendung der zeolithischen Katalysatoren eventuell eine durch Koksabscheidung bedingte Desaktivierung eintritt, empfiehlt es sich, die Zeolithe nach üblichen Methoden zu regenerieren. Durch partielle Verkokung (pre-coke) ist es möglich, die Aktivität des Katalystors für ein Selektivitätsoptimum des gewünschten Reaktionsproduktes einzustellen.

Um eine möglichst hohe Selektivität, hohen Umsatz sowie lange Standzeiten zu erreichen, kann es vorteilhaft sein, die Zeolithe zu modifizieren. Derartige Modifizierungen sind ausführlich beschrieben in DE-A-35 13 725, DE-A-35 46 372, DE-A-36 30 614 und DE-A-36 38 010.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft.

Weitere Katalysatoren für das erfindungsgemäße Verfahren sind Phosphate, insbesondere Aluminiumphosphate, Silciumaluminiumphoshate, Siliciumeisenaluminiumphosphate, Cobaltaluminiumphopshate, Cerphosphat, Zirkonphosphate, Borphosphat, Eisenphosphat, Strontiumphosphate oder deren Gemische.

Als Aluminiumphosphat-Katalysatoren werden für das erfindungsgemäße Verfahren insbesondere unter hydrothermalen Bedingungen synthetisierte Aluminiumphosphate eingesetzt, die Zeolithstruktur besitzen. Die unter hydrothermalen Bedingungen hergestellten Aluminiumphosphate sind z.B. APO-5, APO-9, APO-11, APO-12, APO-14, APO-21, APO-25, APO-31 und APO-33. Synthesen dieser Verbindungen sind in EP-A-132 708, US-A-4 310 440 und US-A-4 473 663 beschrieben.

Die für das erfindungsgemäße Verfahren eingesetzten Siliciumaluminiumphosphate sind z.B. SAPO-5, SAPO-11, SAPO-31 und SAPO-34. Die Synthese dieser Verbindung wird z.B. in EP-A-103 117, US-A-3 440 871 beschrieben.

Auf diese Phosphate können auch Imprägnierung (Tränken und Aufsprühen) oder in manchen Fällen auch durch Ionenaustausch Modifizierungskomponenten, wie voran bei Zeolithen beschrieben, aufgebracht werden. Auch kann wie bei den Zeolithkatalysatoren eine Modifizierung mit Säuren erfolgen.

Geeignete saure Katalysatoren sind z.B. auch die sauer wirkenden Oxide von Elementen der III. und IV. Hauptgruppe sowie der IV. bis VI. Nebengruppe des periodischen Systems, insbesondere Oxide wie Siliciumdioxid in Form von Kieselgel, Kieselgur, Quarz, weiterhin Titandioxid, Zirkondioxid, Phosphoroxide, Vanadiumoxide, Nioboxide, Boroxide, Chromoxide, Molybdänoxider, Wolframoxide oder Bims oder Gemische dieser Oxide. Auch können diese Oxide durch Aufbringung von Modifizierungskomponenten, wie voran bei den Zeolithkatalysatoren beschrieben, dotiert werden. Die Behandlung mit Säuren, wie voran bei den Zeolithkatalysatoren beschrieben, ist ebenfalls eine eventuelle Möglichkeit der Modifizierung.

Auch mit Phosphorsäure oder Borsäure getränkte Katalysatoren können eingesetzt werden. Phosphorsäure oder Borsäure wird auf $SiO_2$-, $Al_2O_3$-, $TiO_2$- oder Bims-Träger z.B. durch Auftränken oder Versprühen aufgebracht.

Als Katalysatoren eignen sich oxidische Katalysatoren wie z.B. Siliciumdioxid, phosphorsäure- oder schwefelsäuregetränkte und kalzinierte Kieselsäurekatalysatoren und Oxide, Mischoxide, Phosphate oder Silicate der Elemente Bor, Aluminium, Titan und Zirkon wie z.B. Borosilikate, Borphosphate, Aluminiumsilikate, Aluminiumphosphate, Aluminiumtitanate. Sehr günstige Eigenschaften haben diejenigen Mischoxide, welche zeolithische Strukturen aufweisen. In manchen Fällen ist Kieselsäure ein ausreichend saurer Katalysator.

Die bevorzugte Flüssigphasenreaktion kann beispielsweise als Suspensions-, Riesel- oder Sumpfreaktion bei Temperaturen von 180 bis 300 °C und Drücken von 0,01 bis 50 bar, vorzugsweise 200 bis 280 °C und 0,02 bis 25 bar, besonders bevorzugt bei 220 bis 260 °C und 0,02 bis 15 bar durchgeführt werden. Als flüssiges Reaktionsmedium eignen sich unter den Reaktionsbedingungen inerte hochsiedende Flüssigkeiten mit Siedepunkten von 150 bis 500 °C/1 bar, vorzugsweise 180 bis 450 °C/1 bar, z.B. Weißöl, Decalin, Tetralin, Diphenylether, Silikonöle und besonders Vakuumrückstandsöl.

Eine bevorzugte Ausführungsform besteht darin, daß man das Ausgangsmaterial in das erhitzte Reaktionsmedium pumpt, wobei man die als Katalysator benötigte Säure entweder bereits im Lösungsmittel vorlegt oder zusammen mit dem umzusetzenden Material zuführt.

Als saure Katalysatoren eignen sich die für die Gasphasenreaktion genannten Katalysatoren sowie anorganische Säuren wie z.B. Schwefelsäure und Phosphorsäure oder organische Sulfonsäuren wie Benzolsulfonsäure, p-Toluolsulfonsäure und Dodecylbenzolsulfonsäure.

Man kann auch zusammen mit dem Ausgangsmaterial einen Strom von gasförmigem Chlorwasserstoff, Bromwasserstoff oder Schwefeltrioxid in das Reaktionsmedium einleiten. Ebenso kann man als Lewissäuren wirkende Metallverbindungen wie beispielsweise Eisen-, Aluminium-, Bor-, Zink-, Titan-, Zirkon- oder Zinnverbindungen als Katalysatoren verwenden.

Nach der Umsetzung werden die entstandenen Produkte durch übliche Verfahren, aus dem Reaktionsgemisch isoliert. Beispielsweise können die gasförmigen Reaktionsprodukte sofort in eine Trennung eingebracht und z.B. in einer Fraktionierkolonne in ihre Einzelkomponenten zerlegt werden. Nichtumgesetzte Ausgangsstoffe werden gegebenenfalls in die Umsetzung zurückgeführt.

Die Verbindungen II lassen sich nach üblichen Methoden (Houben/Weyl, 4. Auflage, Bd. 6/3, S. 203-293, 1965) aus entsprechenden Diolen III und Aldehyden IVa oder deren Acetalen IVb nach folgender Reaktionsgleichung herstellen.

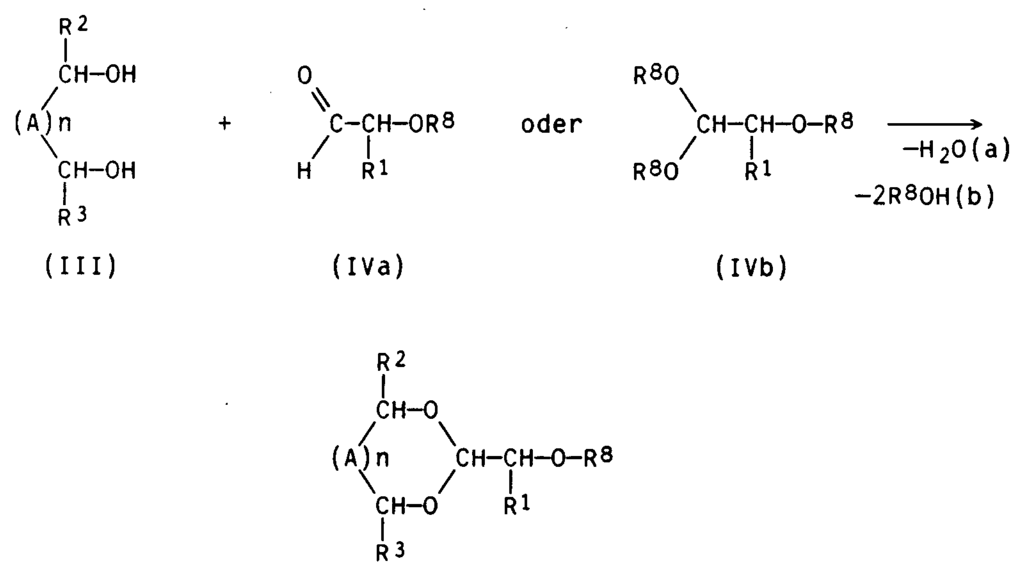


(III) + (IVa) oder (IVb)

(II)

Die Verbindungen III und IVa/b sind z.T. bekannt oder können nach an sich bekannten Methoden (für III: Houben/Weyl, 4. Auflage, Bd. 6/1b, Seite 1-638 (1984) für IVa/b: Houben/Weyl, Bd. E3, Seite 1-608 (1983) hergestellt werden.

Insbesondere bedeuten die Reste $R^1$ bis $R^7$ in den Formeln I bis IV unabhängig voneinander Wasserstoff, unverzweigtes oder verzweigtes $C_1$- bis $C_8$-Alkyl wie Methyl, Ethyl, Propyl, Isopropyl, n-Butyl, iso-Butyl, sec.-Butyl, tert.-Butyl, Pentyl, Hexyl, Heptyl oder Octyl, unverzweigtes oder verzweigtes $C_2$- bis $C_{16}$-Alkoxyalkyl wie z.B. Methoxymethyl, Ethoxymethyl, n- und iso-Propoxymethyl, n- und iso-Butoxymethyl, n- und iso-Hexoxymethyl, n- und iso-Heptoxymethyl, Methoxyethyl, Ethoxyethyl, n- und iso-Propoxyethyl, n- und iso-Butoxyethyl, n- und iso-Pentoxyethyl, n- und iso-Hexoxyethyl, Methoxypropyl, Ethoxypropyl, n- und iso-Propoxypropyl, n- und iso-Butoxypropyl, n- und iso-Pentoxypropyl, Methoxybutyl, Ethoxybutyl, n- und iso-Propoxybutyl, n- und iso-Butoxybutyl, Methoxypentyl, Ethoxypentyl, n- und iso-Propoxypentyl, Methoxyhexyl, Ethoxyhexyl, Methoxyheptyl, Ethoxyheptyl, n- und iso-Propoxyheptyl, Hexoxyheptyl, Methoxyoctyl, Ethoxyoctyl, n- und iso-Butoxyoctyl, Hexoxyoctyl, Octoxyoctyl. Bevorzugt ist $C_2$- bis $C_{10}$-Alkoxyalkyl, besonders bevorzugt $C_2$-$C_8$-Alkoxyalkyl, unverzweigtes oder verzweigtes $C_2$- bis $C_8$-Alkenyl wie Vinyl, Propenyl, Isopropenyl, Butenyl, Isobutenyl, Pentenyl, Hexenyl, Heptenyl und Octenyl, unverzweigtes oder verzweigtes $C_2$-$C_8$-Alkinyl wie Ethinyl, Propinyl, 3-Methylbut-1-in-1-yl, Hexinyl und Hexeninyl, $C_5$- bis $C_8$-Cycloalkyl wie Cyclopentyl, Cyclohexyl, Cycloheptyl und Cyclooctyl, Aryl wie Phenyl und Naphthyl, Phenalkyl oder Alkylphenyl mit 7 bis 10 Kohlenstoffatomen wie Tolyl, Xylyl, Phenethyl oder Benzyl, $C_8$- bis $C_{10}$-Phenalkenyl wie Styryl und Phenylbutenyl, einen Heterocyclus wie einen heterocyclischen oder heteroaromatischen Rest, wobei als Heteroatome insbesondere Sauerstoff neben Stickstoff und Schwefel in Betracht kommen. Die heteroaromatischen Reste haben vorteilhaft 5 oder 6 Ringglieder mit 1 bis 2 Heteroatomen und können einen weiteren 5- oder 6-Ring, der z.B. aromatisch oder heteroaromatisch ist, ankondensiert haben. Beispielsweise seien folgende Reste aufgeführt: Pyrrolyl, Imidazolyl, Pyrazolyl, Triazolyl, Tetrazolyl, Oxazolyl, Isoxazolyl, Thiazolyl und Thiadiazolyl.

Heterocyclische Reste sind z.B. gesättigte oder Doppelbindungen enthaltende heterocyclische Reste mit 1 oder 2 Heteroatomen wie Pyrrolidinyl, Thiazolinyl, Thiazolidinyl, Piperidinyl, Morpholinyl, Dihydrofuranyl, Tetrahydrofuranyl, Dihydropyranyl, Tetrahydropyranyl, Dioxolanyl, Dioxanyl, Thioxanyl, Tetrahydrothiophenyl, Tetrahydrothiopyranyl und Dithianyl.

Weiterhin können die Reste $R^2$ und $R^3$, $R^2$ und $R^4$, $R^3$ und $R^4$, $R^2$ und $R^6$, $R^3$ und $R^6$, $R^4$ und $R^5$ oder $R^6$ und $R^7$ gemeinsam einen weiteren aliphatischen oder heteroaliphatischen Cyclus schließen.

Der bifunktionelle Rest A bedeutet

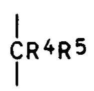

oder

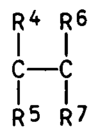

und

der Index n bedeutet 0 oder 1.

Der Rest $R^8$ in den Verbindungen II und IVa und b bedeutet Wasserstoff oder unverzweigtes oder verzweigtes $C_1$- bis $C_{12}$-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, tert.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, bevorzugt unverzweigtes oder verzweigtes $C_1$-$C_4$-Alkyl besonders bevorzugt Methyl, Ethyl und n-Butyl.

Die Reste $R^1$ bis $R^7$ können zusätzliche Substituenten tragen, sofern diese Substituenten unter den Reaktionsbedingungen inert sind. Hierzu gehören z.B. die Halogene Fluor, Chlor, Brom und Iod, Nitrogruppen, Cyangruppen, Thiol- und Thioethergruppen, Sulfon- und Sulfoxidgruppen, Estergruppen wie Methylester, Ethylester oder Butylester, Amidgruppen wie unsubtituiertes Amid oder N-mono-substituiertes bzw. N,N-disubstituiertes Amid wie N-Methylamid, N-Ethylamid, N-Propylamid, N,N-Dimethylamid, N,N-Diethylamid oder N-Methyl-N-Butylamid und auch cycische bzw. aromatische Derivate wie Morpholid oder Anilid sowie insbesondere Alkoxygruppen wie Methoxy, Ethoxy, n- und iso-Propoxy, n- und iso-Butoxy und auch weiter substituierte Reste wie z.B. 2-(Methoxy)-ethoxy oder 2-Chlorethoxy.

Unter den Verbindungen I sind diejenigen neu, in denen A für $CR^4R^5$, n für 1 und $R^1$ für Wasserstoff steht.

Die cyclischen 6- bis 8-gliedrigen Vinylen-1,2-dioxyverbindungen I sind wertvolle Zwischenprodukte zur Herstellung von pharmakologisch wirksamen Verbindungen, Wirkstoffen für den Pflanzenschutz und Kunststoffen.

Beispiele

Beispiel 1

Ein Gemisch aus 380 g (5 Mol) 1,3-Propandiol, 479 g (5,5 Mol) 85 %igem Methoxiacetaldehyd und 250 ml Cyclohexan wurde mit 5 g Ionenaustauscher Lewasorb® AC 10 (Bayer Aktiengesellschaft) solange am Wasserauskreiser erhitzt, bis sich kein Wasser mehr abschied. Der Ionenaustauscher wurde abfiltriert, das Lösungsmittel im Vakuum abgezogen und der Rückstand im Vakuum fraktioniert. Man erhielt 587 g 2-Methoximethyl-1,3-dioxan mit einem Siedebereich von 104 bis 115 °C/150 mbar, entsprechend 89 % Ausbeute.

In einem Rührkolben wurden 250 g Vakuumgasöl mit 2,5 g Dodecylbenzolsulfonsäure bei 250 °C vorgelegt und Stickstoff eingeleitet. Man pumpte 587 g 2-Methoximethyl-1,3-dioxan mit einer Geschwindigkeit von 20 ml/h zu und destillierte die Reaktionsprodukte gleichzeitig ab. Das Rohdestillat wurde anschließend destillativ aufgearbeitet und ergab neben Methanol 258 g 1,4-Dioxep-2-en mit Sdp. = 73 bis 80 °C/200 mbar sowie 182 g nicht verbrauchtes Ausgangsmaterial, entsprechend einem Umsatz an 2-Methoximethyl-1,3-dioxan von 69 % und einer Selektivität von 84 %.

Beispiel 2

Ein Gemisch aus 520 g (5 Mol) 2,2-Dimethyl-1,3-propandiol, 479 g (5,5 Mol) 85 %igem Methoxiacetaldehyd und 250 ml Cyclohexan wurde mit 5 g Ionenaustauscher Lewasorb AC 10 solange am Wasserauskreiser erhitzt, bis sich kein Wasser mehr abschied. Der Ionenaustauscher wurde abfiltriert, das Lösungsmittel im Vakuum abgezogen und der Rückstand im vakuum fraktioniert. Man erhielt 632 g 2-Methoximethyl-5,5-dimethyl-1,3-dioxan mit einem Siedepunkt von 96 °C/60 mbar, entsprechend 79 % Ausbeute.

In einem Rührkolben wurden 250 g Vakuumgasöl mit 2,5 g Dodecylbenzolsulfonsäure bei 250 °C vorgelegt und Stickstoff eingeleitet. Man pumpte 600 g 2-Methoximethyl-5,5-dimethyl-1,3-dioxan mit einer Geschwindigkeit von 20 ml/h zu und destillierte die Reaktionsprodukte gleichzeitig ab. Das Rohdestillat wurde anschließend destillativ aufgearbeitet und ergab neben Methanol 213 g 6,6-Dimethyl-1,4-dioxep-2-en mit Sdp. = 65 bis 67 °C/100 mbar sowie 258 g nicht verbrauchtes Ausgangsmaterial, entsprechend einem Umsatz an 2-Methoximethyl-5,5-dimethyl-1,3-dioxan von 57 % und einer Selektivität von 78 %.

Beispiel 3

Ein Gemisch aus 590 g (5 Mol) 2-Ethyl-2-methyl-1,3-propandiol, 479 g (5,5 Mol) 85 %igem Methoxiacetaldehyd und 500 ml Chloroform wurde mit 10 g Pyridinium-p-Toluolsulfonat solange am Wasserauskreiser erhitzt, bis sich kein Wasser mehr abschied. Die verbleibende Lösung wurde mit einem Mischbettionenaustauscher entsalzt, das Lösungsmittel i. Vak. entfernt und der Rückstand destilliert. Man erhielt 774 g 2-Methoximethyl-5-ethyl-5-methyl-1,3-dioxan mit Sdp. 58 °C/1 mbar, entsprechend 89 % Ausbeute.

In einem Rührkolben wurden 250 g Vakuumgasöl mit 2,5 g Dodecylbenzolsulfonsäure bei 250 °C vorgelegt und Stickstoff eingeleitet. Man pumpte 774 g 2-Methoximethyl-5-ethyl-5-methyl-1,3-dioxan mit einer Geschwindigkeit von 20 ml/h zu und destillierte die Reaktionsprodukte gleichzeitig ab. Das Rohdestillat wurde anschließend destillativ aufgearbeitet und ergab neben Methanol 297 g 6-Ethyl-6-methyl-1,4-dioxep-2-en mit Sdp. = 119 bis 121 °C/400 mbar sowie 356 g nicht verbrauchtes Ausgangsmaterial, entsprechend einem Umsatz an 2-Methoximethyl-5-ethyl-5-methyl-1,3-dioxan von 54 % und einer Selektivität von 71 %.

Beispiel 4

Ein Gemisch aus 670 g (5 Mol) 2-Methoximethyl-2-methyl-1,3-propandiol, 479 g (5,5 Mol) 85 %igem Methoxiacetaldehyd und 250 ml Chloroform wurde mit 10 g Pyridinium-p-Toluolsulfonat solange am Wasserauskreiser erhitzt, bis sich kein Wasser mehr abschied. Die Lösung wurde mit einem Mischbettionenaustauscher entsalzt, das Lösungsmittel im Vakuum abgezogen und der Rückstand im Vakuum fraktioniert. Man erhielt 864 g 2,5-Dimethoximethyl-5-methyl-1,3-dioxan mit Sdp. 95 °C/10 mbar, entsprechend 91 % Ausbeute.

In einem Rührkolben wurden 500 g Vakuumgasöl mit 5 g Dodecylbenzolsulfonsäure bei 250 °C vorgelegt und Stickstoff eingeleitet. Man pumpte 150 g 2,5-Dimethoximethyl-5-methyl-1,3-dioxan mit einer Geschwindigkeit von 30 ml/h zu und destillierte die Reaktionsprodukte gleichzeitig ab. Das Rohdestillat wurde anschließend destillativ aufgearbeitet und ergab neben Methanol 23,3 g 6-Methoximethyl-6-methyl-1,4-dioxep-2-en mit Sdp. = 101 bis 105 °C/80 mbar sowie 85 g nicht verbrauchtes Ausgangsmaterial, entsprechend einem Umsatz an 2,5-Dimethoximethyl-5-methyl-1,3-dioxan von 43 % und einer Selektivität von 43 %.

Beispiel 5

Zu einem siedenden Gemisch aus 392 g (4 Mol) 2-Isopropylacrolein, 1280 g (40 Mol) Methanol und 720 g (8,8 Mol) 30 %igem Formaldehyd tropfte man innerhalb 30 Minuten 352 g (4,4 Mol) 50 %ige Natronlauge und rühre anschließend weitere 60 Minuten unter Rückfluß. Dann wurde eingeengt, Methylisobutylketon zugegeben und das Restwasser azeotrop ausgekreist. Ausgefallenes Natriumformiat wurde abfiltriert und das Filtrat destilliert. Man erhielt 504 g 2-Methoximethyl-2-isopropyl-1,3-propandiol vom Siedepunkt 96 bis 100 °C/1 mbar, entsprechend 78 % Ausbeute.

Ein Gemisch aus 486 g (3 Mol) 2-Methoximethyl-2-isopropyl-1,3-propandiol, 287 g (3,3 Mol) 85 %igem Methoxiacetaldehyd und 250 ml Cyclohexan wurde mit 5 g Ionenaustauscher Lewasorb AC 10 solange am Wasserauskreiser erhitzt, bis sich kein Wasser mehr abschied. Der Ionenaustauscher wurde abfiltriert, das Lösungsmittel im Vakuum abgezogen und der Rückstand im Vakuum fraktioniert. Man erhielt 543 g 2,5-Dimethoximethyl-5-isopropyl-1,3-dioxan mit Sdp. 95 °C/1 mbar, entsprechend 83 % Ausbeute.

In einem Rührkolben wurden 250 g Vakuumgasöl mit 3,75 g Dodecylbenzolsulfonsäure bei 230 °C vorgelegt und Stickstoff eingeleitet. Man pumpte 543 g 2,5-Dimethoximethyl-5-isopropyl-1,3-dioxan mit einer Geschwindigkeit von 20 ml/h zu und destillierte die Reaktionsprodukte gleichzeitig ab. Das Rohdestillat wurde anschließend destillativ aufgearbeitet und ergab neben Methanol 109 g 6-Methoximethyl-6-isopropyl-1,4-dioxep-2-en mit Sdp. = 117 bis 118 °C/45 mbar sowie 331 g nicht verbrauchtes Ausgangsmaterial, entsprechend einem Umsatz an 2,5-Dimethoximethyl-5-isopropyl-1,3-dioxan von 39 % und einer Selektivität von 60 %.

Beispiel 6

Ein Gemisch aus 288 g (2 Mol) 1,1-Dimethylolcyclohexan, 191 g (2,2 Mol) 85 %igem Methoxiacetaldehyd und 250 ml Cyclohexan wurde mit 2 g p-Toluol-Sulfonsäure solange am Wasserauskreiser erhitzt, bis sich kein Wasser mehr abschied. Die erhaltene Lösung wurde mit verdünnter Natronlauge gewaschen, das Lösungsmittel im Vakuum abgezogen und der Rückstand im Vakuum fraktioniert. Man erhielt 388 g 3-Methoximethyl-2,4-dioxaspiro[5.5]undecan mit Sdp. 95 °C/1 mbar, entsprechend 97 % Ausbeute.

In einem Rührkolben wurden 250 g Vakuumgasöl mit 2,5 g Dodecylbenzolsulfonsäure bei 250 °C vorgelegt und Stickstoff eingeleitet. Man pumpte 3-Methoximethyl-2,4-dioxaspiro[5.5]undecan mit einer Geschwindigkeit von 20 ml/h zu und destillierte die Reaktionsprodukte gleichzeitig ab. Das Rohdestillat wurde anschließend destillativ aufgearbeitet und ergab neben Methanol 83 g 2,5-Dioxaspiro[6.5]dodec-3-en mit Sdp. = 117 °C/30 mbar sowie 268 g nicht verbrauchtes Ausgangsmaterial, entsprechend einem Umsatz an 3-Methoximethyl-2,4-dioxaspiro[5.5]undecan von 31 % und einer Selektivität von 82 %.

Beispiel 7

Die Mischung bestehend aus 152 g (2 Mol) 1,2-Propandiol, 148 g (2 Mol) 85 %igem Methoxiacetaldehyd und 400 ml Cyclohexan wurde mit 1 g Pyridinium-p-Toluolsulfonat versetzt und solange am Wasserauskreiser unter Rückfluß erhitzt, bis sich kein Wasser mehr abschied. Die gewonnene Lösung wurde mit Wasser gewaschen, das Lösungsmittel wurde im Vakuum abgezogen und der Rückstand im Vakuum fraktioniert. Man erhielt 245 g 2-Methoximethyl-4-methyl-1,3-dioxolan mit Sdp. 79 bis 82 °C/70 mbar, entsprechend 93 % Ausbeute.

a) Insgesamt 100 g 2-Methoximethyl-4-methyl-1,3-dioxolan wurden bei 350 °C innerhalb 5 Stunden über 20 g eines Trägerkatalysators mit der Zusammensetzung 20 % Phosphorsäure auf Kieselsäure geleitet. Aus dem Kondensat wurde zunächst mit Cyclohexan Methanol ausgekreist und anschließend destilliert. Man erhielt 21,2 g 5-Methyl-1,4-dioxen mit Sdp. 105 °C/1013 mbar, entsprechend 28 % Ausbeute.

b) In einer Rührapparatur wurden 250 g Vakuumgasöl mit 2,5 g Dodecylbenzolsulfonsäure bei 250 °C vorgelegt. Bei gleichzeitigem Abdestillieren der Reaktionsprodukte wurden zusammen mit einem Stickstoffstrom insgesamt 176 g 2-Methoximethyl-5-methyl-1,3-dioxolan innerhalb neun Stunden eingetragen. Aus dem Destillat wurde mit Cyclohexan Methanol ausgekreist und der Rückstand fraktioniert. Man erhielt 113 g 5-Methyl-1,4-dioxen mit Sdp. 105 bis 106 °C/1013 mbar, entsprechend 85 % Ausbeute.

Beispiel 8

Die Mischung bestehend aus 50 g (0,43 Mol) trans-1,2-Cyclohexandiol, 54 g (0,47 Mol) 1,1,2-Trimethoxiethan und 300 ml Cyclohexan wurde mit 2 g Pyridinium-p-Toluolsulfonat versetzt und so lange am Wasserauskreiser unter Rückfluß erhitzt, bis sich kein Methanol mehr abschied. Die gewonnene Lösung wurde mit Wasser gewaschen, das Lösungsmittel wurde im Vakuum abgezogen und der Rückstand im Vakuum fraktioniert. Man erhielt 64 g 8-Methoximethyl-7,9-dioxabicyclo[4.3.0$^{1.6}$]nonan mit Sdp. 67 bis 70 °C/1 mbar, entsprechend 86 % Ausbeute.

Insgesamt 50 g 8-Methoximethyl-7,9-dioxabicyclo[4.3.0$^{1.6}$]nonan wurden bei 300 °C zusammen mit einem Strom von 40 l/h $N_2$ innerhalb 2,5 Stunden über 40 g Kieselsäurestränge geleitet. Die Vakuumdestillation des Kondensats ergab 21 g 2,5-Dioxabicyclo[4.4.0]dec-3-en mit Sdp. 40 bis 43 °C/1 mbar, entsprechend 52 % Ausbeute.

Die folgenden Beispiele veranschaulichen die Erfindung unter Verwendung von Heterogenkatalysatoren in der Gasphase.

Die in den nachfolgenden Beispielen 9 bis 19 eingesetzten Katalysatoren sind:

Katalysator A

Der Borosilikatzeolith des Pentasil-Typs wird in einer hydrothermalen Synthese aus 640 g hochdispersem $SiO_2$, 122 g $H_3BO_3$, 8000 g einer wäßrigen 1,6-Hexandiamin-Lösung (Mischung 50:50 Gew.%) bei 170 °C unter autogenem Druck in einem Rührautoklaven hergestellt. Nach Abfiltrieren und Auswaschen wird das kristalline Reaktionsprodukt bei 100 °C/24 h getrocknet und bei 500 °C/24 h calciniert. Dieser Borosilikatzeolith setzt sich zusammen aus 94,2 Gew.% $SiO_2$ und 2,3 Gew.% $B_2O_3$.

Katalysator A wird erhalten, indem man den Borosilikatzeolith mit einem Verformungshilfsmittel zu 2-mm-Strängen verformt, bei 110 °C/16 h trocknet und bei 500 °C/24 h calciniert.

Katalysator B

Katalysator B wird hergestellt, indem man Katalysator A mit $Ce(NO_3)_2$ dotiert, bei 130 °C/2 h trocknet und bei 540 °C/2 h calciniert. Der Ce-Gehalt berägt 1,8 Gew.%.

Katalysator C

Katalysator C wird wie Katalysator B hergestellt, jedoch mit einer wäßrigen Lösung aus Pd- und Ce-Nitrat anstatt Ce-Nitrat getränkt. Der Pd-GEhalt beträgt 1,3 Gew.%, der Ce-Gehalt 3,6 Gew.%.

Katalysator D

Ein Aluminosilikatzeolith vom Pentasil-Typ wurde unter hydrothermalen Bedingungen bei autogenem Druck und 150 °C aus 65 g hochdispersem $SiO_2$, 20,3 g $Al_2(SO_4)_3$ x 18 $H_2O$ in 1 kg mit wäßrigem 1,6-Hexandiamin-Lösung (Mischung 50:50 Gew.%) in einem Rührautoklaven hergestellt. Nach Abfiltrieren und Auswaschen wurde das kristalline Reaktionsprodukt bei 110 °C/24 Stunden getrocknet und bei 500 °C/24 Stunden calciniert. Dieser Aluminosilikatzeolith enthielt 91,6 Gew.% $SiO_2$ und 4,6 Gew.% $Al_2O_3$.

Der Katalysator wurde mit einem Verformungshilfsmittel zu 2-mm-Strängen verformt, bei 110 °C/16 h getrocknet und bei 500 °C/24 h calciniert.

Katalysator E

Siliciumaluminiumphosphat-5 (SAPO-5) wird aus einer Mischung aus 200 g 98 %iger Phosphorsäure, 136 g Boehmit, 60 g Kieselöl (30 %ig), 287 g Tripropylamin und 587 g $H_2O$. Diese Mischung wird bei 150 °C während 168 h unter autogenem Druck umgesetzt. Nach Filtration wird das kristalline Produkt bei 120 °C getrocknet und bei 500 °C calciniert. SAPO-5 enthält 49,8 Gew.% $P_2O_5$, 33,0 Gew.% $Al_2O_3$, 6,2 Gew.% $SiO_2$. SAPO-5 wird mit einem Verstrangungshilfsmittel zu 3-mm-Strängen verformt, bei 120 °C getrocknet und bei 500 °C calciniert.

Katalysator F

$BPO_4$ wird hergestellt, indem man 49 g $H_3BO_3$ mit 117 g $H_3PO_4$ (75 %ig) in einem Kneter zusammengibt, überschüssiges Wasser abdampft und das Umsetzungsprodukt zu 3-mm-Strängen verformt. Diese Stränge werden bei 100 °C getrocknet und bei 350 °C calciniert. Katalysator F enthält 8,77 Gew.% B und 28,3 Gew.% P.

Katalysator G

Katalysator G ist ein gefälltes Aluminiumphosphat, das durch Fällung aus $Al(NO_3)_3$-$H_3PO_4$-Lösung mit $NH_3$ bei pH = 6 bis 7 erhalten wird. Nach Abfiltrieren des Niederschlags wird bei 110 °C getrocknet und bei 500 °C calciniert. Katalysator G enthält 28,5 Gew.% Al und 13,2 Gew.% P.

Katalysator H

$CePO_4$ wird durch Fällung aus 52 g $Ce(NO_3)_3$ x 6 $H_2O$ und 56 g $NaH_2PO_4$ x 2 $H_2O$ erhalten. Nach der Filtration wird das Material zu Strängen verformt, bei 120 °C getrocknet und bei 450 °C calciniert. Katalysator H enthält 47,1 Gew.% Ce und 12,7 Gew.% P.

Katalysator I

$SiO_2$ im Handel erhältlich unter D 11-10®.

Katalysator J

$Al_2O_3$ im Handel erhältlich unter D 10-10®.

Katalysator K

D 10-10® wird mit $H_3BO_3$ imprägniert, getrocknet bei 110 °C und bei 500 °C/5h calciniert. Der Katalysator T setzt sich zusammen aus 85 % $Al_2O_3$ und 15 % $B_2O_3$.

Beispiele 9 bis 19

Die Reaktionen in der Gasphase wurden unter isothermen Bedingungen in einem Rohrreaktor (Wendel, 0,6 cm Innendurchmesser, 90 cm Länge) mindestens 6 Stunden lang durchgeführt. Die Reaktionsprodukte wurden durch übliche Methoden abgetrennt und charakterisiert. Die quantitative Bestimmung der Reaktionsprodukte und der Ausgangsprodukte erfolgte gaschromatographisch.

Die genauen Reaktionsbedingungen und Ergebnisse sind in der nachfolgenden Tabelle 1 zusammengestellt.

Tabelle 1: 1,4-Diazabicyclo-2,2,2-octan (DABCO) aus Piperazin

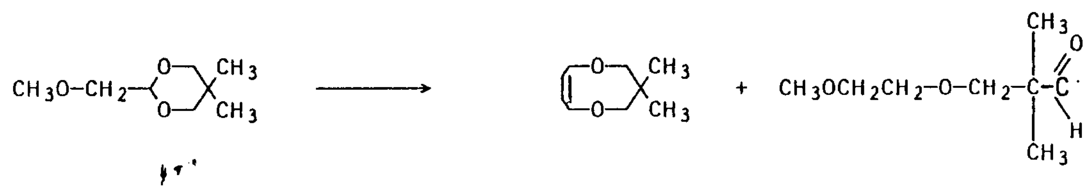

| Beispiel | 9 | 10 | 11 | 12 | 13 | 14 | 15 | 16 | 17 | 18 | 19 |
|---|---|---|---|---|---|---|---|---|---|---|---|
| Katalysator | A | B | C | D | E | F | G | H | I | J | K |
| Temp., °C | 300 | 350 | 350 | 300 | 350 | 350 | 350 | 350 | 350 | 350 | 350 |
| WHSV $h^{-1}$ | 2,9 | 3,0 | 3,0 | 3,0 | 2,3 | 3,3 | 2,3 | 2,3 | 3,0 | 3,0 | 3,0 |
| Umsatz % des Edukts | 61,3 | 95,4 | 95,5 | 75,2 | 100 | 36,4 | 78,2 | 33,3 | 66,3 | 25,8 | 43,8 |
| Selekt. % des Diox...... | 53,0 | 61,7 | 61,3 | 49,1 | 41,0 | 95,1 | 38,4 | 71,2 | 43,2 | 37,2 | 35,0 |
| Selekt. % der offenkettigen Verbindung | 33,3 | 21,5 | 23,3 | 29,5 | 42,3 | 3,5 | 49,6 | - | 55,5 | 50,6 | 57,6 |

Beispiel 20

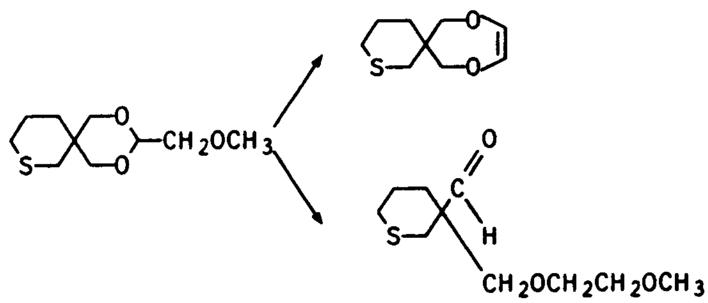

Im Katalysator A wird bei 350 °C und einer Belastung von 2,4 g einer 50 %igen THF-Lösung der Verbindung I pro g Katalysator und Stunde ein Umsatz von 36 % erzielt. Die Selektivität des Dioxependerivates beträgt 41 %, die des Aldehydethers 14 %. Bei 300 °C unter sonst gleichen Reaktionsbedingungen werden 8,2 % Umsatz und 58 % Selektivität für das Dioxependerivat gefunden.

Beispiel 21

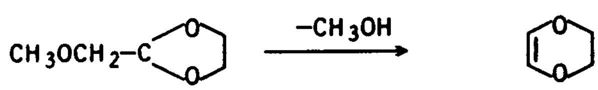


Die Umsetzung des Methoxyacetaldehydglykolacetat (gelöst in THF 50:50) erfolgt am Borphosphat-Katalysator bei 350 °C und WHSV = 3 $h^{-1}$ vollständig, die Selektivität an Dihydrodioxan beträgt 58 %.

Beispiel 22

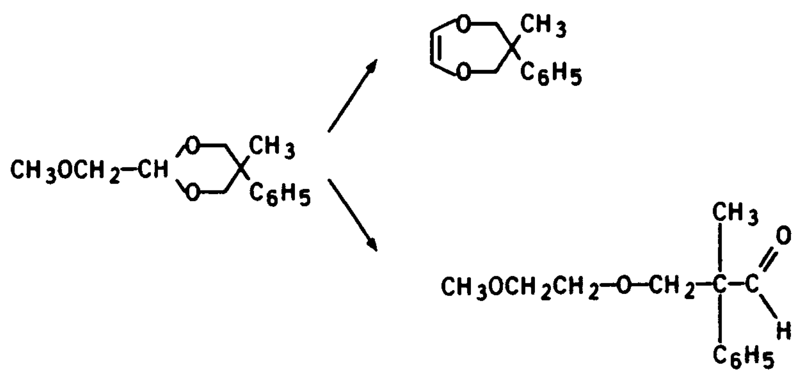

Die Umsetzung von Methoxyacetaldehyd-2-methyl-2-phenyl-propandiolacetal erfolgt bei 300 °C und WHSV = 3 $h^{-1}$ zu 20 %. Die Selektivität an Dioxependerivat beträgt 56,5 %.

An Katalysator D findet man unter gleichen Reaktionsbedingungen 30 % Umsatz und 26,5 % Selektivität für das Dioxependerivat bzw. 20,3 % für die offenkettige Verbindung.

## Patentansprüche

1. Verfahren zur Herstellung cyclischer 6- bis 8-gliedriger Vinylen-1,2-dioxyverbindungen der allgemeinen Formel I

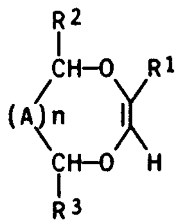

(I)

in der
A die Reste

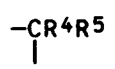 oder 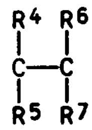 ,

| | |
|---|---|
| $R^1$ bis $R^7$ | unabhängig voneinander Wasserstoff, einen aliphatischen, cycloaliphatischen, aromatischen, aliphatisch-aromatischen oder heterocyclischen Rest oder gemeinsam $R^2$ und $R^3$, $R^2$ und $R^4$, $R^3$ und $R^4$, $R^2$ und $R^6$, $R^3$ und $R^6$, $R^4$ und $R^5$ oder $R^6$ und $R^7$ einen cycloaliphatischen oder heterocyclischen Ring, wobei die Reste $R^1$ bis $R^7$ gegebenenfalls noch unter den Reaktionsbedingungen inerte Substituenten tragen, und |
| n | 0 oder 1 bedeuten, |

dadurch gekennzeichnet, daß man cyclische 2-Oxymethyl-1,3-dioxaverbindungen oder deren Derivate der allgemeinen Formel II

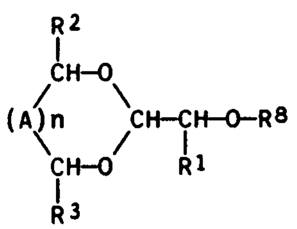

(II),

in der $R^1$ bis $R^7$, A und n die obengenannten Bedeutungen haben und $R^8$ für Wasserstoff oder $C_1$- bis $C_{12}$-Alkyl steht, in Gegenwart von sauren Katalysatoren bei Temperaturen von 150 bis 450 °C und Drücken von 0,001 bis 50 bar umsetzt.

2. Verfahren gemäß Anspruch 1, dadurch gekennzeichnet, daß man Ausgangsverbindungen der Formel I verwendet, in denen $R^1$ bis $R^7$ unabhängig voneinander Wasserstoff, $C_1$- bis $C_8$-Alkyl, $C_2$- bis $C_{16}$-Alkoxyalkyl, $C_2$- bis $C_8$-Alkenyl, $C_2$- bis $C_8$-Alkinyl, $C_5$- bis $C_8$-Cycloalkyl, Aryl, $C_7$- bis $C_{10}$-Alkylphenyl, $C_7$- bis $C_{10}$-Phenalkyl, $C_8$- bis $C_{10}$-Phenalkenyl, einen Heterocyclus bedeutet.

3. 1,4-Dioxep-2-ene der allgemeinen Formel I

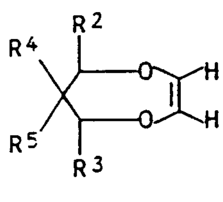

(I),

in der
$R^2$ bis $R^5$ die in Anspruch 1 genannten Bedeutungen haben.

## Claims

**1.** A process for the preparation of a cyclic 6-membered to 8-membered vinylene-1,2-dioxy compound of the formula I

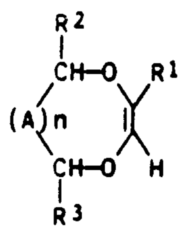

(I)

where A is

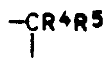

or

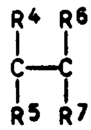

,

$R^1$ to $R^7$ independently of one another are each hydrogen or an aliphatic, cycloaliphatic, aromatic, aliphatic-aromatic or heterocyclic radical, or $R^2$ and $R^3$, $R^2$ and $R^4$, $R^3$ and $R^4$, $R^2$ and $R^6$, $R^3$ and $R^6$, $R^4$ and $R^5$ or $R^6$ and $R^7$ together form a cycloaliphatic or heterocyclic ring, and $R^1$ to $R^7$ may furthermore carry substituents which are inert under the reaction conditions, and n is 0 or 1, wherein a cyclic 2-oxymethyl-1,3-dioxa compound or one of its derivatives of the formula II

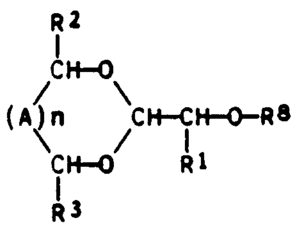

(II)

where $R^1$ to $R^7$, A and n have the abovementioned meanings, and $R^8$ is hydrogen or $C_1$-$C_{12}$-alkyl, is converted in the presence of an acidic catalyst at from 150 to 450 °C and under from 0.001 to 50 bar.

**2.** A process as claimed in claim 1, wherein a starting compound of the formula I where $R^1$ to $R^7$ independently of one another are each hydrogen, $C_1$-$C_8$-alkyl, $C_2$-$C_{16}$-alkoxyalkyl, $C_2$-$C_8$-alkenyl, $C_2$-$C_8$-alkynyl, $C_5$-$C_8$-cycloalkyl, aryl, $C_7$-$C_{10}$-alkylphenyl, $C_7$-$C_{10}$-phenalkyl, $C_8$-$C_{10}$-phenalkyl or a heterocyclic structure is used.

**3.** A 1,4-dioxep-2-ene of the formula I

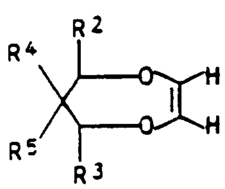

(I)

where $R^2$ to $R^5$ have the meanings stated in claim 1.

## Revendications

1. Procédé de préparation de composés du type vinylène-1,2-dioxycycliques, hexagonaux ou octagonaux, de la formule générale I

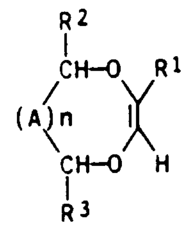

dans laquelle
A représente les radicaux

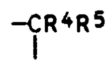 ou 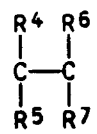

$R^1$ à $R^7$ représentent chacun, indépendamment les uns des autres, un atome d'hydrogène, un radical aliphatique, cycloaliphatique, aromatique, aliphatique-aromatique, ou hetérocyclique, ou bien, $R^2$ et $R^3$, $R^2$ et $R^4$, $R^3$ et $R^4$, $R^2$ et $R^6$, $R^3$ et $R^6$, $R^4$ et $R^5$, ou $R^6$ et $R^7$, forment, en commun, un noyau cycloaliphatique ou hétérocyclique, où les radicaux $R^1$ à $R^7$ peuvent éventuellement encore porter des substituants inertes dans les conditions réactionnelles et

n est égal à O ou à 1,

caractérisé en ce que l'on fait réagir des composés du type 2-oxyméthyl-1,3-dioxacycliques, ou leurs dérivés, de la formule générale II

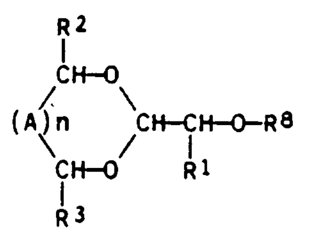

(II)

dans laquelle les symboles $R^1$ à $R^7$, A et n possèdent chacun les significations qui leur ont été attribuées ci-dessus et $R^8$ représente un atome d'hydrogène ou un radical alkyle en $C_1$ à $C_{12}$, en présence de catalyseurs acides, à des températures de 150 à 450 °C et sous des pressions de 0,001 à 50 bars.

2. Procédé suivant la revendication 1, caractérisé en ce que l'on utilise des composés de départ de la formule I dans laquelle les symboles $R^1$ à $R^7$ représentent chacun, indépendamment les uns des autres, un atome d'hydrogène, un radical alkyle en $C_1$ à $C_8$, alcoxyalkyle en $C_2$ à $C_{16}$, alcényle en $C_2$ à $C_8$, alcynyle en $C_2$ à $C_8$, cycloalkyle en $C_5$ à $C_8$ aryle, alkylphényle en $C_7$ à $C_{10}$, phénalkyle en $C_7$ à $C_{10}$, phénalcényle en $C_8$ à $C_{10}$, un radical hétérocyclique.

**3.** 1,4-Dioxep-2-ènes de la formule générale I

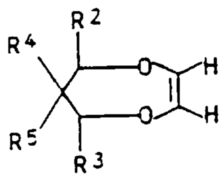

(I)

dans laquelle
les symboles $R^2$ à $R^5$ ont les significations qui leur ont été attribuées dans la revendication 1.